# EUROPEAN PATENT SPECIFICATION

(11) **EP 1 996 711 B1**
(45) Date of publication and mention of the grant of the patent: **05.05.2010**
(21) Application number: 06852018.8
(22) Date of filing: 01.12.2006
(51) Int. Cl.: A61K 39/07

(54) **NOVEL PLANT VIRUS PARTICLES AND METHODS OF INACTIVATION THEREOF**
NEUE PFLANZENVIRUSPARTIKEL UND VERFAHREN ZU DEREN INAKTIVIERUNG
NOUVELLES PARTICULES DE VIRUS DE PLANTE ET PROCÉDÉ D'INACTIVATION DE CELLES-CI

(30) Priority: 02.12.2005 US 742197 P
(43) Date of publication of application: 03.12.2008
(73) Proprietor: Pfenex, Inc., San Diego, CA 92121 (US)
(72) Inventor: RASOCHOVA, Lada, Del Mar, CA 92014 (US); PHELPS, Jamie, P., Aurora, CO (US)
(74) Representative: Ward, Siobhan
(86) International application number: PCT/US2006/046160
(87) International publication number: WO 2008/085147

(56) References cited:
- WO-A-2005/086667
- LANGEVELD J P M ET AL: "Inactivated recombinant plant virus protects dogs from a lethal challenge with canine parvovirus" VACCINE, BUTTERWORTH SCIENTIFIC. GUILDFORD, GB, vol. 19, no. 27, 14 June 2001 (2001-06-14), pages 3661-3670, XP004241784 ISSN: 0264-410X
- BURGE W D ET AL: "EFFECT OF HEAT ON VIRUS INACTIVATION BY AMMONIA" APPLIED AND ENVIRONMENTAL MICROBIOLOGY, vol. 46, no. 2, 1983, pages 446-451, XP002497209 ISSN: 0099-2240 cited in the application

### SEQUENCE LISTING

<110> Rasochova, Lada
   Phelps, Jamie Patrick
<120> Novel Plant virus Particles and Methods of Inactivation Thereof
<130> DOW-200P
<160> 24
<170> PatentIn version 3.3
<210> 1
   <211> 25
   <212> PRT
   <213> Bacillus anthracis
<400> 1
   Ser Asn Ser Arg Lys Lys Arg Ser Thr Ser Ala Gly Pro Thr Val Pro
   1               5                   10                  15
   Asp Arg Asp Asn Asp Gly Ile Pro Asp
               20                  25
<210> 2
   <211> 25
   <212> PRT
   <213> Bacillus anthracis
<400> 2
   Ser Pro Glu Ala Arg His Pro Leu Val Ala Ala Tyr Pro Ile Val His
   1               5                   10                  15
   Val Asp Met Glu Asn Ile Ile Leu Ser
               20                  25
<210> 3
   <211> 25
   <212> PRT
   <213> Bacillus anthracis
<400> 3
   Arg Ile Ile Phe Asn Gly Lys Asp Leu Asn Leu Val Glu Arg Arg Ile
   1               5                   10                  15
   Ala Ala Val Asn Pro Ser Asp Pro Leu
               20                  25
<210> 4
   <211> 26
   <212> PRT
   <213> Bacillus anthracis
<400> 4
   Glu Arg Ile Ile Phe Asn Gly Lys Asp Leu Asn Leu Val Glu Arg Arg
   1               5                   10                  15
   Ile Ala Ala Val Asn Pro Ser Asp Pro Leu
   20 25
<210> 5
   <211> 25
   <212> PRT
   <213> Bacillus anthracis
<400> 5
   Arg Gln Asp Gly Lys Thr Phe Ile Asp Phe Lys Lys Tyr Asn Asp Lys
   1 5 10 15
   Leu Pro Leu Tyr Ile Ser Asn Pro Asn
   20 25
<210> 6
   <211> 21
   <212> PRT
   <213> Bacillus anthracis
<400> 6
   Ser Asp Phe Glu Lys Val Thr Gly Arg Ile Asp Lys Asn Val Ser Pro
   1 5 10 15
   Glu Ala Arg His Pro
   20
<210> 7
   <211> 25
   <212> PRT
   <213> Bacillus anthracis
<400> 7
   His Val Asp Met Glu Asn Ile Ile Leu Ser Lys Asn Glu Asp Gln Ser
   1 5 10 15
   Thr Gln Asn Thr Asp Ser Gln Thr Arg
   20 25
<210> 8
   <211> 25
   <212> PRT
   <213> Bacillus anthracis
<400> 8
   Thr Asp Ser Gln Thr Arg Thr Ile Ser Lys Asn Thr Ser Thr Ser Arg
   1               5                   10                  15
   Thr His Thr Ser Glu Val His Gly Asn
               20                  25
<210> 9
   <211> 26
   <212> PRT
   <213> Bacillus anthracis
<400> 9
   Glu Thr Asp Ser Gln Thr Arg Thr Ile Ser Lys Asn Thr Ser Thr Ser
   1               5                   10                  15
   Arg Thr His Thr Ser Glu Val His Gly Asn
               20                  25
<210> 10
   <211> 25
   <212> PRT
   <213> Bacillus anthracis
<404> 10
<210> 11
   <211> 21
   <212> PRT
   <213> Bacillus anthracis
<400> 11
   Ser Asn Ser Asn Ser Ser Thr Val Ala Ile Asp His Ser Leu Ser Leu
   1               5                   10                  15
   Ala Gly Glu Arg Thr
               20
<210> 12
   <211> 18
   <212> PRT
   <213> Bacillus anthracis
<400> 12
   Glu Thr Met Gly Leu Asn Thr Ala Asp Thr Ala Arg Leu Asn Ala Asn
   1 5 10 15
   Ile Arg
<210> 13
   <211> 24
   <212> PRT
   <213> Bacillus anthracis
<400> 13
   Glu Pro Thr Thr Ser Leu Val Leu Gly Lys Asn Gln Thr Leu Ala Thr
   1 5 10 15
   Ile Lys Ala Lys Glu Asn Gln Glu
   20
<210> 14
   <211> 24
   <212> PRT
   <213> Bacillus anthracis
<400> 14
   Pro Ser Lys Asn Leu Ala Pro Ile Ala Leu Asn Ala Gln Asp Asp Phe
   1 5 10 15
   Ser Ser Thr Pro Ile Thr Met Asn
   20
<210> 15
   <211> 20
   <212> PRT
   <213> Bacillus anthracis
<400> 15
   Ser Glu Val Leu Pro Gln Ile Gln Glu Thr Thr Ala Arg Ile Ile Phe
   1 5 10 15
   Asn Gly Lys Asp
   20
<210> 16
   <211> 25
   <212> PRT
   <213> Bacillus anthracis
<400> 16
<21.0> 17
   <211> 25
   <212> PRT
   <213> Bacillus anthracis
<400> 17
<210> 18
   <211> 23
   <212> PRT
   <213> Bacillus anthracis 5
<400> 18
   Gln Gly Lys Asp Ile Thr Glu Phe Asp Phe Asn Phe Asp Gln Gln Thr
   1               5                   10                  15
   Ser Gln Asn Ile Lys Asn Gln
               20
<210> 19
   <211> 20
   <212> PRT
   <213> Bacillus anthracis
<400> 19
   Asp Arg Asn Asn Ile Ala Val Gly Ala Asp Glu Ser Val Val Lys Glu
   1 5 10 15
   Ala His Arg Glu
   20
<210> 20
   <211> 25
   <212> PRT
   <213> Bacillus anthracis
<400> 20
   Arg Glu Val Ile Asn Ser Ser Thr Glu Gly Leu Leu Leu Asn Ile Asp
   1 5 10 15
   Lys Asp Ile Arg Lys Ile Leu Ser Gly
   20 25
<210> 21
   <211> 19
   <212> PRT
   <213> Bacillus anthracis
<400> 21
   Asp Met Leu Asn Ile Ser Ser Leu Arg Gln Asp Gly Lys Thr Phe Ile
   1 5 10 15
   Asp Phe Lys
<210> 22
   <211> 22
   <212> PRT
   <213> Bacillus anthracis
<400> 22
   Thr Lys Glu Asn Thr Ile Ile Asn Pro Ser Glu Asn Gly Asp Thr Ser
   1 5 10 15
   Thr Asn Gly Ile Lys Lys
   20
<210> 23
   <211> 764
   <212> PRT
   <213> Bacillus anthracis
<400> 23
   Met Lys Lys Arg Lys Val Leu Ile Pro Leu Met Ala Leu Ser Thr Ile
   1               5                   10                  15
   Leu Val Ser Ser Thr Gly Asn Leu Glu Val Ile Gln Ala Glu Val Lys
               20                  25                  30
   Gln Glu Asn Arg Leu Leu Asn Glu Ser Glu Ser Ser Ser Gln Gly Leu
           35                  40                  45
   Leu Gly Tyr Tyr Phe Ser Asp Leu Asn Phe Gln Ala Pro Met Val Val
       50                  55                  60
   Thr Ser Ser Thr Thr Gly Asp Leu Ser Ile Pro Ser Ser Glu Leu Glu
   65                  70                  75                  80
   Asn Ile Pro Ser Glu Asn Gln Tyr Phe Gln Ser Ala Ile Trp Ser Gly
                   85                  90                  95
   Phe Ile Lys Val Lys Lys Ser Asp Glu Tyr Thr Phe Ala Thr Ser Ala
               100                 105                 110
   Asp Asn His Val Thr Met Trp Val Asp Asp Gln Glu Val Ile Asn Lys
           115                 120                 125
   Ala Ser Asn Ser Asn Lys Ile Arg Leu Glu Lys Gly Arg Leu Tyr Gln
       130                 135                 140
   Ile Lys Ile Gln Tyr Gln Arg Glu Asn Pro Thr Glu Lys Gly Leu Asp
   145                 150                 155                 160
   Phe Lys Leu Tyr Trp Thr Asp Ser Gln Asn Lys Lys Glu Val Ile Ser
                   165                 170                 175
   Ser Asp Asn Leu Gln Leu Pro Glu Leu Lys Gln Lys Ser Ser Asn Ser
               180                 185                 190
   Arg Lys Lys Arg Ser Thr Ser Ala Gly Pro Thr Val Pro Asp Arg Asp
           195                 200                 205
   Asn Asp Gly Ile Pro Asp Ser Leu Glu Val Glu Gly Tyr Thr Val Asp
       210                 215                 220
   Val Lys Asn Lys Arg Thr Phe Leu Ser Pro Trp Ile Ser Asn Ile His
   225                 230                 235                 240
   Glu Lys Lys Gly Leu Thr Lys Tyr Lys Ser Ser Pro Glu Lys Trp Ser
   245 250 255
   Thr Ala Ser Asp Pro Tyr Ser Asp Phe Glu Lys Val Thr Gly Arg Ile
   260 265 270
   Asp Lys Asn Val Ser Pro Glu Ala Arg His Pro Leu Val Ala Ala Tyr
   275 280 285
   Pro Ile Val His Val Asp Met Glu Asn Ile Ile Leu Ser Lys Asn Glu
   290 295 300
   Asp Gln Ser Thr Gln Asn Thr Asp Ser Glu Thr Arg Thr Ile Ser Lys
   305 310 315 320
   Asn Thr Ser Thr Ser Arg Thr His Thr Ser Glu Val His Gly Asn Ala
   325 330 335
   Glu Val His Ala Ser Phe Phe Asp Ile Gly Gly Ser Val Ser Ala Gly
   340 345 350
   Phe Ser Asn Ser Asn Ser Ser Thr Val Ala Ile Asp His Ser Leu Ser
   355 360 365
   Leu Ala Gly Glu Arg Thr Trp Ala Glu Thr Met Gly Leu Asn Thr Ala
   370 375 380
   Asp Thr Ala Arg Leu Asn Ala Asn Ile Arg Tyr Val Asn Thr Gly Thr
   385 390 395 400
   Ala Pro Ile Tyr Asn Val Leu Pro Thr Thr Ser Leu Val Leu Gly Lys
   405 410 415
   Asn Gln Thr Leu Ala Thr Ile Lys Ala Lys Glu Asn Gln Leu Ser Gln
   420 425 430
   Ile Leu Ala Pro Asn Asn Tyr Tyr Pro Ser Lys Asn Leu Ala Pro Ile
   435 440 445
   Ala Leu Asn Ala Gln Asp Asp Phe Ser Ser Thr Pro Ile Thr Met Asn
   450 455 460
   Tyr Asn Gln Phe Leu Glu Leu Glu Lys Thr Lys Gln Leu Arg Leu Asp
   465 470 475 480
   Thr Asp Gln Val Tyr Gly Asn Ile Ala Thr Tyr Asn Phe Glu Asn Gly
   485 490 495
   Arg Val Arg Val Asp Thr Gly Ser Asn Trp Ser Glu Val Leu Pro Gln
   500 505 510
   Ile Gln Glu Thr Thr Ala Arg Ile Ile Phe Asn Gly Lys Asp Leu Asn
           515                 520                 525
   Leu Val Glu Arg Arg Ile Ala Ala Val Asn Pro Ser Asp Pro Leu Glu
       530                 535                 540
   Thr Thr Lys Pro Asp Met Thr Leu Lys Glu Ala Leu Lys Ile Ala Phe
   545                 550                 555                 560
   Gly Phe Asn Glu Pro Asn Gly Asn Leu Gln Tyr Gln Gly Lys Asp Ile
                   565                 570                 575
   Thr Glu Phe Asp Phe Asn Phe Asp Gln Gln Thr Ser Gln Asn Ile Lys
               580                 585                 590
   Asn Gln Leu Ala Glu Leu Asn Ala Thr Asn Ile Tyr Thr Val Leu Asp
           595                 600                 605
   Lys Ile Lys Leu Asn Ala Lys Met Asn Ile Leu Ile Arg Asp Lys Arg
       610                 615                 620
   Phe His Tyr Asp Arg Asn Asn Ile Ala Val Gly Ala Asp Glu Ser Val
   625                 630                 635                 640
   Val Lys Glu Ala His Arg Glu Val Ile Asn Ser Ser Thr Glu Gly Leu
                   645                 650                 655
   Leu Leu Asn Ile Asp Lys Asp Ile Arg Lys Ile Leu Ser Gly Tyr Ile
               660                 665                 670
   Val Glu Ile Glu Asp Thr Glu Gly Leu Lys Glu Val Ile Asn Asp Arg
           675                 680                 685
   Tyr Asp Met Leu Asn Ile Ser Ser Leu Arg Gln Asp Gly Lys Thr Phe
       690                 695                 700
   Ile Asp Phe Lys Lys Tyr Asn Asp Lys Leu Pro Leu Tyr Ile Ser Asn
   705                 710                 715                 720
   Pro Asn Tyr Lys Val Asn Val Tyr Ala Val Thr Lys Glu Asn Thr Ile
                   725                 730                 735
   Ile Asn Pro Ser Glu Asn Gly Asp Thr Ser Thr Asn Gly Ile Lys Lys
               740                 745                 750
   Ile Leu Ile Phe Ser Lys Lys Gly Tyr Glu Ile Gly
           755                 760
<210> 24
   <211> 22
   <212> PRT
   <213> Bacillus anthracis
<400> 24
   Ser Leu Leu Thr Glu Val Glu Thr Pro Ile Arg Asn Glu Gly Cys Arg
   1               5                   10                  15
   Cys Asn Asp Ser Ser Asp
               20

## Description

### FIELD OF THE INVENTION

The present invention relates generally to a method of inactivating plant viruses, produced by plants, for use as vaccines and the like. More specifically, the present invention relates to virus inactivation methods.

### BACKGROUND OF THE INVENTION

Vaccination can protect individuals and entire populations from infectious agents. Developing safe and effective vaccines is, however, not always straightforward for a number of reasons ranging from identification of effective antigens to safety concerns with developed vaccines. The use of viruses as carriers of foreign peptides has been explored in the field of composite virus vaccines. Such vaccines are based on chimeric viruses, which are hybrids of different animal virus components. Usually the major component of such hybrids is derived from a virus that which is or has been rendered harmless, and the minor component is a selected antigenic component of a pathogenic virus. For example, a pox virus such as vaccinia or an attenuated poliovirus may be used as a vector for immunogenic components of other animal viruses including human viruses.

However, such techniques as discussed above can be disadvantages. Such vaccines are produced from viruses grown in cell culture systems, which can be expensive to design and run. The composite virus approach involves genetic manipulation of live, animal-infecting viruses, with the risk that mutations may give rise to novel forms of the virus with altered infectivity, antigenicity, and/or pathogenicity. In addition, the animal virus used as the vector can be a virus to which the animal may already have been exposed, and the animal may already be producing antibodies to the vector. Thus, the vector can be destroyed by the immune system before the incorporated antigenic site of the second virus induces an immune response.

A number of methods have been used for mammalian virus inactivation. These include: UV irradiation, UV/psoralen irradiation, Pentose Pharmaceuticals chemicals, Microwaves, Formalin, BPL, pH, temperature, and incubation in ammonium chloride. UV irradiation has been used to inactivate recombinant plant viruses. *See e.g.* Langeveld et al. (2001) "Inactivated Recombinant Plant Virus Protects Dogs from a Lethal Challenge with Canine Parvovirus," Vaccine 19:3661-3670.

Patents that relate to methods of producing the particles and to the use of the particles, particularly as vaccines include U.S. Patent No. 6,110,466, which discusses assembled particles of a plant virus containing a predetermined foreign peptide as part of the coat protein of the virus and U.S. Patent No. 6,884,623 which discusses assembled particles of a plant virus containing a foreign peptide insert in the coat protein of the virus, where the site of the insert is preferably free from direct sequence repeats flanking the insert.

U.S. Patent No. 5,602,242 relates to recombinant RNA viruses for encapsidation of genetically engineered viral sequences in heterologous, preferably rod-shaped coat, protein capsids. This patent also relates to methods of making and using such recombinant viruses, specifically with respect to the transfection of plants to bring about genotypic and phenotypic changes in the plants. Means for deleting or inactivating viral coat protein genes were described in Ahlquist et al. (1981) "Complete Nucleotide Sequence of Brome Mosaic Virus RNA3," J. Mol. Biol. 153:23-38.

Burge et al., "Effect of Heat on Virus Inactivation by Ammonia", Appl. Environ. Microbiology, Aug 46(2):446-51, 1983, discusses the effect of heat on virus inactivation with ammonium chloride. Bacteriophage f2 and poliovirus 1 (an enveloped, mammalian virus) were studied. Temperatures above 40°C were found to damage the virus tested herein. Cramer WN, et al. "Kinetics of virus inactivation by ammonia", Appl Environ Microbiology, Mar 45(3):760-5, 1983, like Burge *et al.,* used ammonium chloride, at a range of pHs, to treat sewage in an attempt to inactivate viruses. Again, bacteriophage f2 and poliovirus 1 (strain CHAT) were studied. The results of those tests are reported to show that the poliovirus inactiviation rate was influenced much less, if at all, by the effect of NH₄⁺ concentration than was the inactivation rate of f2. The paper discusses possible applications of the methodology in waste water treatment plants as a possible alternative to chlorine, particularly for members of the enterovirus group.

### BRIEF SUMMARY OF THE INVENTION

The present invention includes methods for inactivating a plant virus by administering ammonium sulfate to plant material selected from the group consisting of plants, plant tissue, plant cells and protoplasts at a pH above 8.0 to produce an inactivated virus-like particle (VLP); incubating the plant material for at least ten hours; and then harvesting the inactivated VLP from the plant material. These methods can include the incorporation of a foreign peptide into the virus. The virus can be in a non-enveloped RNA virus. The inactivated VLP can presents a heterologous bioactive peptide. The ammonium sulfate is administered at a concentration of 0.5M to 1.0M., generally at 0.7M. The pH is generally 9.0 and the plant material can be incubated at room temperature. The VLP is non-infectious because it lacks at least a portion of RNA present in the plant virus. Additionally, it can not initiate infection upon inoculation and is incapable of replicating. The virus like particle and the vaccines are not within the scope conferred by the present application

The present invention can also include the inactivation of chimeric plant virus particles and integration of the inactivation step into the virus particle purification procedure. The inactivation method renders the virus incapable of infecting plants. The integrity of virus particle is maintained while the infectious viral genomic RNA that is present inside the virus particle is destroyed. These methods can be scalable and can be integrated into the purification process.

The present invention also includes methods of producing a non-infectious VLP by administering ammonium sulfate to plant material selected from the group consisting of plants, plant tissue, plant cells and protoplasts and lacks at least a portion of RNA present in a plant virus at a pH above 8.0; incubating the plant material for at least ten hours; and harvesting the inactivated VLP from the plant material, wherein the VLP is not capable of replicating.

### BRIEF DESCRIPTION OF THE FIGURES

**Figure 1** shows RNA extracted from PA10 active and a PA10 inactivated virus run on 1.2 % agarose gel stained with ethidium bromide illustrating CPMV genomic RNA 1 and 2 in the active virus and degraded RNA in the inactive virus preparation.
**Figure 2** illustrates an AIEC chromatogram of PA7E.
**Figures 3-5** demonstrate RNA inactivation for PA9, PA11 and PA18.
**Figure 6** shows the SDS-PAGE gel of a 5 day temperature stability assay for PAIS.
**Figure 7** illustrates anti-PA antibodies in CPMV-PA immunized monkeys as detected by ELISA.
**Figure 8** shows anti-PA antibodies (IgG) in serum and bronchial lavage on day 140.

### BRIEF DESCRIPTION OF THE SEQUENCES

**SEQ ID NO:1** is the peptide sequence of Epitope PA1 used according to Example 3 of the present invention.

**SEQ ID NO:2** is the peptide sequence of Epitope PA2 used according to Example 3 of the present invention.

**SEQ ID NO:3** is the peptide sequence of Epitope PA3 used according to Example 3 of the present invention.

**SEQ ID NO:4** is the peptide sequence of Epitope PA3E used according to Example 3 of the present invention.

**SEQ ID NO:5** is the peptide sequence of Epitope PA4 used according to Example 3 of the present invention.

**SEQ ID NO:6** is the peptide sequence of Epitope PA5 used according to Example 3 of the present invention.

**SEQ ID NO:7** is the peptide sequence of Epitope PA6 used according to Example 3 of the present invention.

**SEQ ID NO:8** is the peptide sequence of Epitope PA7 used according to Example 3 of the present invention.

**SEQ ID NO:9** is the peptide sequence of Epitope PA7E used according to Example 3 of the present invention.

**SEQ ID NO:10** is the peptide sequence of Epitope PA8 used according to Example 3 of the present invention.

**SEQ ID NO:11** is the peptide sequence of Epitope PA9 used according to Example 3 of the present invention.

**SEQ ID NO:12** is the peptide sequence of Epitope PA10 used according to Example 3 of the present invention.

**SEQ ID NO:13** is the peptide sequence of Epitope PA11 used according to Example 3 of the present invention.

**SEQ ID NO:14** is the peptide sequence of Epitope PA12 used according to Example 3 of the present invention.

**SEQ ID NO:15** is the peptide sequence of Epitope PA13 used according to Example 3 of the present invention.

**SEQ ID NO:16** is the peptide sequence of Epitope PA14 used according to Example 3 of the present invention.

**SEQ ID NO:17** is the peptide sequence of Epitope PA15 used according to Example 3 of the present invention.

**SEQ ID NO:18** is the peptide sequence of Epitope PA 16 used according to Example 3 of the present invention.

**SEQ ID NO:1**9 is the peptide sequence of Epitope PA17 used according to Example 3 of the present invention.

**SEQ ID NO:20** is the peptide sequence of Epitope PA18 used according to Example 3 of the present invention.

**SEQ ID NO:21** is the peptide sequence of Epitope PA19 used according to Example 3 of the present invention.

**SEQ ID NO:22** is the peptide sequence of Epitope PA20 used according to Example 3 of the present invention.

**SEQ ID NO:23** is the amino acid sequence of the protective antigen (PA) of the present anthrax vaccine.

**SEQ ID NO:24** is the amino acid sequence of the influenza virus epitope M2e.

### DETAILED DESCRIPTION OF THE INVENTION

The present invention relates in part to novel virus inactivation methods for making novel plant virus-like particles for use as vaccines and the like. Methods for virus inactivation are describe herein. The present invention provides examples of inactivation of chimeric plant virus particles and integration of the inactivation step into the virus particle purification procedure. The inactivation method renders the virus incapable of infecting plants. Embodiments of the present invention can include means and methods to produce a safe vaccine based on an epitope display of epitopes derived from a pathogenic agent on the surface of inactivated plant virus-like particles.

Embodiments of the present invention include an efficient and scalable procedure for inactivation of viruses to produce virus-like particles (VLPs) that lack the full infectious genome of the virus. Such embodiments include using an ammonium sulfate buffer, generally at pH 9, as the initial extraction buffer. Ammonium sulfate is regarded as being non-toxic and acceptable by the regulatory authorities.

Embodiments of the present invention include viral inactivation with ammonium sulfate in a pH range of approximately 9.0. The viral inactivation can occur through RNA cleavage and degradation. The viral particles can become permeabilized allowing for the entrance of ammonium ions into the virus. Therefore, the incubation with ammonium ions should be carried out at pH above 8.0 because at this pH level the virus "swells" which "opens" its structure, thus allowing penetration of the small molecules through the viral coat.

Embodiments of the present invention include methods for the integration of inactivation of particles in a seamless way with other purification operations. These methods can include instances wherein plant tissue is collected and homogenized in an extraction buffer, wherein the buffer is 0.7 M ammonium sulfate at pH 9 and incubated at room temperature for about 20 hrs. After the incubation, the particles are no longer infectious to plants and cannot initiate infection upon inoculation. The same conditions at pH 7.0 did not inactivate virus and higher temperatures *i.e.* 40°C appeared to damage the virus structure.

### Additional Process Steps and Parameters

Embodiments of the present invention can also include milling/homogenizing the plant material in the inactivation buffer, incubating the milled slurry in an inactivation buffer to degrade viral genomic RNA, and purifying the resulting virus particles. The milled material can be further clarified by centrifugation/filtration prior to incubation in the inactivation buffer. After the incubation, the particles can be precipitated by PEG or by increasing the molarity of ammonium sulfate to a level that causes the particle to precipitate from the solution. Buffer exchange and chromatography steps usually follow the inactivation step. The inactivation step can be integrated at any point into the purification procedure. For example, in some procedures, the inactivation was integrated into the process in a following manner: CPMV binding to HIC column in 0.7 M (NH₄)₂SO₄ , pH 7 ► Washing bound CPMV with 0.7 M (NH₄)₂SO₄ , pH 9 ► Elution of CPMV with 0.7 M (NH₄)₂SO₄, pH 9. The following ranges can be utilized: 0.5-1.0 M (NH₄)₂SO₄, pH above 8.0 and temperature between 10 to 40°C.

### Types and Selection of Viruses That Can Be Used to Make VLPs to be treated in the method of the invention

Vaccines can be in the form of antigens and fused to coat proteins of non-enveloped RNA viruses (+, -, and/or double stranded). or plant RNA viruses along with icosahedral plant RNA viruses. Although cowpea mosaic virus is exemplified herein, the methods of the present invention can be applied to other similar viruses. For example, some preferred viruses, for use according to the present invention, are:

| **Table 1.** | | | |
|---|---|---|---|
| **Name** | **Acronym** | **Genus** | **Family** |
| Cowpea chlorotic mottle virus | CCMV | *Bromovirus* | Bromoviridae |
| Cowpea mosaic virus | CPMV | *Comovirus* | Comoviridae |
| Tomato bushy stunt virus | TBSV | *Tombusvirus* | Tombusviridae |
| Alfalfa mosaic virus | AMV | *Alfamovirus* | Bromoviridae |
| Brome mosaic virus | BMV | *Bromovirus* | Bromoviridae |
| Southern bean mosaic virus | SBMV | *Sobemovirus* | Tombusviridae |

The present invention can be applied to any RNA plant virus. To demonstrate this system, the plant virus cowpea mosaic comovirus (CPMV) was chosen. The three-dimensional structure of the CPMV is known, which allows for identification of sites suitable for modification without disruption of the particle structure. To date, viruses from at least nine plant virus genera and three subgroup 2 ssRNA satellite viruses have had their tertiary and quaternary structures solved at high resolution. Some of these are listed above in **Table 1.**

One exemplified group of plant viruses for use as vectors are those whose coat proteins have a β-barrel structure. An advantage of the use of viruses that have a β-barrel structure is that the loops between the individual strands of β-sheet provide convenient sites for the insertion of foreign peptides. Modification of one or more loops can be one strategy for the expression of foreign peptides in accordance with the present invention. Insertions in other regions of the coat protein are also possible, such as insertions into the N-terminus and/or C-terminus.

All plant viruses possessing icosahedral symmetry whose structures have been solved conform to the eight stranded β-barrel fold as exemplified by CPMV, and it is likely that this represents a common structure in all icosahedral viruses. All such viruses are suitable for use in the method of inactivation according to the invention and which viruses may present foreign peptide sequences, which can occur in the loops between the β-strands and/or in the N-terminus and/or C-terminus.

Methods of modifying DNA sequences to insert heterologous or foreign sequences are well known to the art. Generally the viral RNA sequence is converted to a full-length cDNA transcripts and cloned into a vector, then modified by inserting a foreign DNA segment in a region able to tolerate such insertion without disrupting RNA replication, particle formation, or disturbing infectivity.

Comoviruses are a group of at least fourteen plant viruses which predominantly infect legumes. Their genomes consist of two molecules of single-stranded, positive-sense RNA of different sizes which are separately encapsidated in isometric particles of approximately 28 nm diameter. The two types of nucleoprotein particles are termed middle (M) and bottom (B) component as a consequence of their behaviour in cesium chloride density gradients, the RNAs within the particles being known as M and B RNA, respectively. Both types of particle have an identical protein composition, consisting of 60 copies each of a large (VP37) and a small (VP23) coat protein. In addition to the nucleoprotein particles, comovirus preparations contain a variable amount of empty (protein-only) capsids which are known as top (T) component.

In the case of the type member of the comovirus group, cowpea mosaic virus (CPMV), it is known that both M and B RNA are polyadenylated and have a small protein (VPg) covalently linked to their 5' terminus. More limited studies on other comoviruses suggest that these features are shared by the RNAs of all members of the group. Both RNAs from CPMV have been sequenced and shown to consist of 3481 (M) and 5889 (B) nucleotides, excluding the poly (A) tails (van Wezenbeek et al. 1983; Lomonossoff and Shanks, 1983). Both RNAs contain a single, long open reading frame. Expression of the viral gene products occurs through the synthesis and subsequent cleavage of large precursor polypeptides. Both RNAs are required for infection of whole plants. The larger B RNA is capable of independent replication in protoplasts, though no virus particles are produced in this case (Goldbach et al., 1980). This observation, coupled with earlier genetic studies, established that the coat proteins are encoded by M RNA, and the formation of infectious virus particles is dependent on the presence of both B and M viral genomic RNAs.

An advantage of the Comoviridae is that their capsid contains sixty copies each of 3 different β-barrels which can be individually manipulated. All other virus families and genera listed above have similar 3-dimensional structures but with a single type of β-barrel. (In the case of CPMV, for example, the foreign insert can be made immediately preceding the proline 23 (Pro²³) residue in the βB-βC loop of the small capsid protein (VP23). See U.S. Patent No. 6,884,623.

The methd of the present invention can also be applied to icosahedral plant viruses (including those containing β-barrel structures) whose crystal structures have not yet been determined. Where significant sequence homology within the coat protein genes exists between one virus whose crystal structure is unknown and a second virus whose crystal structure is known, alignment of the primary structures will allow the locations of the loops between the β-strands to be inferred [see Dolja, V. V. and Koonin, E. V. (1991) J. Gen. Virol., 72, pp 1481-1486]. In addition, where a virus has only minimal coat protein sequence homology to those viruses whose crystal structure has been determined, primary structural alignments may be used in conjunction with appropriate secondary and tertiary structural prediction algorithms to allow determination of the location of potential insertion sites.

CPMV and bean pod mottle virus (BPMV) shows that the 3-D structures of BPMV and CPMV are very similar and are typical of the Comoviridae in general.

CPMV comprises two subunits, the small (S) and the large (L) coat proteins, of which there are 60 copies of each per virus particle. Foreign peptide sequences may be expressed from either the L or S proteins or from both coat proteins on the same virion.

CPMV is biparite RNA virus. In order to manipulate the genome of any RNA virus to express foreign peptides, cDNA clones of the RNA can be used. Full length cDNA clones of both CPMV RNA molecules are available, which can be manipulated to insert oligonucleotide sequences encoding a foreign peptide. cDNA clones of the genome from plant RNA viruses can be used to generate *in vitro* transcripts that are infectious when inoculated onto plants.

cDNA clones of CPMV RNAs M and B have been constructed, in which the cDNA clone of the M RNA contains an inserted oligonucleotide sequence encoding a foreign peptide, which make use of the cassava vein mosaic (CsVMV) promoter sequence linked to the 5' ends of the viral cDNAs to generate infectious transcripts in the plant. This technique overcomes some of the problems encountered with the use of transcripts generated *in vitro* and is applicable to all plant RNA viruses.

Other viruses can include various bromoviruses, in particular the cowpea chlorotic mottle virus (CCMV) and the sobemoviruses, in particular the southern bean mosaic virus (SBMV). An RNA segment of a tripartite virus can also be used. Examples of such useful viruses are the tripartite viruses of Bromoviridae, such as brome mosaic virus (BMV) and cowpea chlorotic mottle virus (CCMV), which are packaged in icosahedral capsids.

The genome of BMV is divided among messenger sense RNA's 1, 2 and 3 of 3.2, 2.9 and 2.1 kb respectively. The coat protein is encoded by subgenomic RNA 4 that is formed from RNA3. In order for cells to be infected with BMV RNA3, the proteins encoded by BMV RNA's 1 and 2 must be present. These three BMV RNA's are separately encapsidated into identical particles. Each particle contains 180 coat protein. The coat protein can be modified to carry peptide insertions.

The coat proteins of a number of the viruses indicated in **Table 1** has been compared. The similarity of the secondary structural elements and their spatial organization is illustrated in FIG. **10** of United State Patents No: 6,884,623. Any of the loops that lie between the β**-**strands can be used for insertion of foreign epitopes. However, the insertions are made such that the additions are exposed on either the internal or external surface of the virus and such that assembly of the coat protein subunits and the infectivity of the virus are not abolished. The choice of a particular loop can be made using knowledge of the structure of individual coat protein subunits and their interactions with each other, as indicated by the crystal structure, such that any insertions are unlikely to interfere with virus assembly. The choice of precise insertion site can be made, initially, by inspection of the crystal structure, followed by *in vivo* experimentation to identify the optimum site.

Thus, the three dimensional structure of a plant virus can be examined in order to identify portions of a coat protein that are particularly exposed on the virus surface and are therefore potentially good sites for insertion. The amino acid sequence of the exposed portion of a coat protein can also be examined for amino acids that break α-helical structures, because these are also potentially good sites for insertion. Examples of suitable amino acids are proline and hydroxyproline, which in a polypeptide chain interrupt the α-helix and create a rigid kink or bend in the structure. N- and C-termini of coat protein are also attractive sites for insertions.

### Types of Antigens and Epitopes

The present inactivation methods can be applied not only to particles displaying antigenic epitopes that are then used as vaccines but also to particles that display any other useful peptides such as targeting peptides, antimicrobial peptides, and the like. This technology can be also applied to the wild type or modified particles that are then used for covalent linkage of various moieties to the particle surface. This includes linkage of proteins including antigenic proteins, peptides, carbohydrates, lipids, nucleic acids, detection agents (such as fluorescent dyes), radioactive agents, targeting ligands, and the like. The particle complexes can be used as vaccines as well as for delivery of the associated agents to targeted tissues and the like. This technology can be also applied prior to encapsulation of various agents, such as drugs, foreign nucleic acids for expression of foreign genes, toxins, and the like inside the particles that are then used for administration and delivery of the encapsulated agent.

Following are examples that illustrate procedures for practicing the invention. These examples should not be construed as limiting. All percentages are by weight and all solvent mixture proportions are by volume unless otherwise noted.

### EXAMPLES

### Example 1 - Summary of Inactivation of CPMV Particles

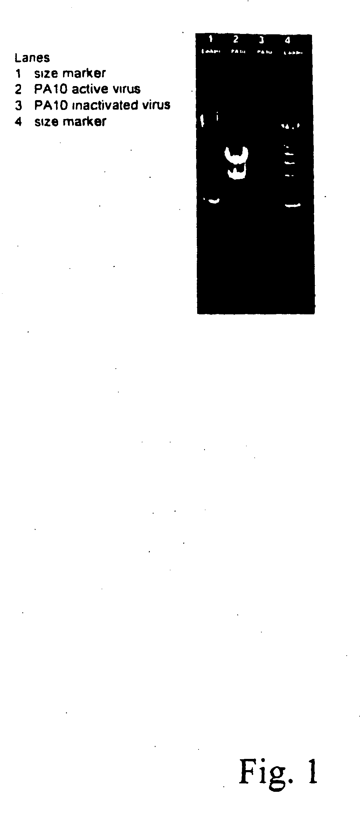

More than 16 different CPMV particles (carrying different epitopes) as well as wild type CPMV virus were inactivated using this procedure. RNA has been isolated from inactivated viruses and run on a gel to determine if the RNA is degraded. The inactivated particles have also been inoculated to plants to test for the ability to induce infection. None of the inoculated plants produced viral infection. RNA isolated from inactivated virus particles was degraded in every case tested (see Figure 1 as an example).

### Example 2 - Further Examples of CPMV Inactivation

A study was set up to determine whether 0.8 M ammonium sulfate can inactivate CPMV while preserving its integrity. 0.8 M ammonium sulfate was used as part of the present purification process. Increase in pH during the process would permealize the virus but would also increase the concentration of free NH₃.

The conclusion from this study was that 0.8 M ammonium sulfate at pH 9 and at pH 7 both at 22°C and 40°C preserved virus integrity and that the virus infectivity was lost only at pH 9. The control experiments where CPMV was incubated in 30 mM Tris-HCI at 22 and 40°C produced fully infective CPMV particles. From these results, it was further concluded that it is combination of 0.8 M ammonium sulfate and pH 9 which is required to cause inactivation and not temperature or ammonium sulfate alone. The experiments in this study were carried out on a milled cell sap adjusted to appropriate ammonium sulfate concentration and pH and there was a concern that a compound in the plant slurry was causing inactivation (e.g. psoralens). To prove or disprove this, a second study was set up to determine if purified CPMV particles can be inactivated using combination of ammonium sulfate and pH 9. Various purity grade chemicals were used to determine if some impurities in the chemicals were responsible for inactivation. An experimental matrix was set-up and all conditions tested as shown in Table 1. 0.7 M ammonium sulfate was used as it was part of our re-optimized process so that an easy integration was possible.

| **Table 2.** An experimental matrix for (NH₄)₂SO₄ inactivation study at 22 °C for 20 h. | | | |
|---|---|---|---|
| **No.** | **Purity (NH₄)₂SO4** | **Concentration (NH₄)₂SO4** | **30mM Tris-HCl** |
| 1 | ANALAR | 0.1 | Yes |
| 2 | ARISTAR | 0.5 | No |
| 2 | | 0.7 | |

The concentrations of 0.5 M and 0.7 M (NH₄)₂SO at pH 9 inactivated CPMV while the 0.1 M (NH₄)₂SO₄ at pH 9 did not. Figure **1** shows RNA, extracted from active and inactivated virus, run on 1.2 % agarose gel and stained with EtBr. The results show presence of CPMV genomic RNA 1 and 2 in the active virus, and degraded RNA in the inactive virus preparation. The same results have been obtained for over 15 other chimeric viral particles and for the wild type virus.

### Example 3 - Chimeric CPMV particles used in the inactivation experiments.

Chimeric CPMV particles were engineered to express peptides derived from the protective antigen ("PA") protein of *Bacillus anthracis.* The peptides were expressed on the large and/or small coat proteins of CPMV, using the methods described in the US patents 5,874,087, 5,958,422, and 6,110,466. The following peptides were expressed:

| **Table 3.** | | |
|---|---|---|
| **Epitope** | **Peptide sequence** | **SEQ ID NO:** |
| PA1 | SNSRKKRSTSAGPTVPDRDNDGIPD | 1 |
| PA2 | SPEARHPLVAAYPIVHVDMENIILS | 2 |
| PA3 | RIIENGKDLNLVERRIAAVNPSDPL | 3 |
| PA3E | ERIIFNGKDLNLVERRIAAVNPSDPL. | 4 |
| A4 | RQDGKTFIDFKKYNDKLPLYISNPN | 5 |
| PA5 | SDFEKVTGRIDKNVSPEARHP | 6 |
| PA6 | HVDMENMLSKNEDQSTQNTDSQTR | 7 |
| PA7 | TDSQTRTISKNTSTSRTHTSEVHGN | 8 |
| PA7E | ETDSQTRTISKNTSTSRTHTSEVHGN | 9 |
| PA8 | HGNAEVHASFFDIGGSVSAGFSNSN | 10 |
| PA9 | SNSNSSTVAIDHSLSLAGERT | 11 |
| PA10 | ETMGLNTADTARLNANIR | 12 |
| PA11 | EPTTSLVLGKNQTLATIKAKENQE | 13 |
| PA12 | PSKNLAPIALNAQDDFSSTPITMN | 14 |
| PA13 | SEVLPQIQETTARIIFNGKD | 15 |
| PA14 | NGKDLNLVERRIAAVNPSDPLETTK | 16 |
| PA15 | ETTKPDMTLKEALKIAFGFNEPNGN | 17 |
| PA16 | QGKDITEFDFNFDQQTSQNIKNQ | 18 |
| PA17 | DRNNIAVGADESVVKEAHRE | 19 |
| PA18 | REVINSSTEGLLLNIDKDIRKILSG | 20 |
| PA19 | DMLNISSLRQDGKTFIDFK | 21 |
| PA20 | TKENTIINPSENGDTSTNGIKK | 22 |

### Example 4 - Production of Chimeric CPMV Particles in Plants.

Cowpea California #5 seeds from Ferry Morse, part number 1450, were germinated over night at room temperature in wet paper towels. Germinated seeds were transferred into soil. Seven days post germination the seedlings were inoculated with WT or chimeric CPMV particles. After inoculation, the plants were grown at 25°C with a photo period of 16 hours light and 8 hours dark for two to three weeks. The leaves that showed symptoms were harvested and frozen at -80°C prior to purification.

### Example 5 - Inactivation of Chimeric CPMV Particles and Purification of Inactivated Chimeric CPMV Virus Like Particles

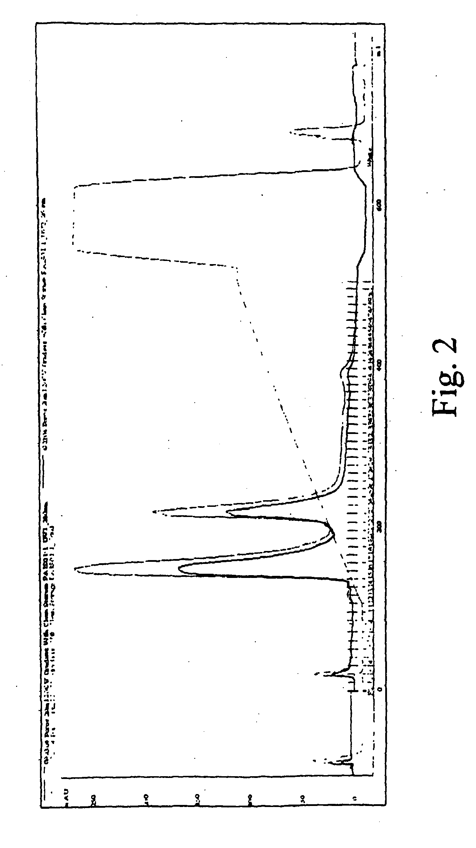

40g of CPMV infected leaf tissue was frozen at -80°C. The frozen leaf tissue was crushed by hand and poured into a Waring high speed blender, part number 8011S. 120ml of cold inactivation buffer (0.5M ammonium sulfate, 0.03M Tris base pH 9.00, 0. 2mM PMSF) was poured onto the crushed leaves. The leaves were ground 2 times for 3 seconds at high speed. The solution was decanted into a 500ml centrifuge bottle. The blender was washed with 30ml of cold inactivation buffer and the wash was poured into a 500ml centrifuge bottle. The solution was centrifuged at 15,000G for 30 minutes to remove the plant cellular debris. The supernatant was decanted into a graduated cylinder and incubated to inactivate the virus for 20 hours at room temperature. To precipitate the CPMV virus, cold PEG 6000 solution (20% PEG 6000, 1M NaCl) was added to the supernatant to bring the final PEG concentration to 4% PEG 6000 with 0.2M NaCl, and the solution was gently mixed. The solution was allowed to precipitate for 1 hour on ice. The virus precipitate solution was then centrifuged at 15,000G for 30 minutes to collect the CPMV virus pellet. The supernatant was poured off and the virus was immediately resuspended in anion exchange binding buffer (30mM Tris base, pH 7.50). To further purify the virus like particles, the protein mixture was fractionated by anion exchange chromatography using POROS 50 HQ strong anion exchange resin from Applied Biosystems, part number 1-2559-11. The 20 column volume gradient was from buffer A, 30mM Tris base, pH 6.75, to buffer B, 30mM Tris base, pH 6.75 with 1M NaCl. The chromatography was run with an AKTAexplorer from Amersham Biosciences, part number 18-1112-41. **Figure 2** illustrates the AIEC chromatogram of PA7E. All samples listed in the Example 3 were processed using the method described in this Example with similar results. Two major peaks were detected. The blue trace is the absorbance at 280, the red trace is the absorbance at 260, the green trace is the percent buffer B, and the brown trace is the conductivity. The red ticks on the bottom of the chromatogram are the fractions. The first peak on the gradient, which contained the desired virus like particles, was buffer exchanged into PBS buffer, pH 7.4 using a 100 kDa cutoff membrane Millipore spin concentrator, part number UFC910096. The samples were then stored at -80° C. The second peak contained the cleaved particle contaminate. An SDS-PAGE gel was prepared, with the PA7E PEG precipitate AIEC load, WT CPMV standard, and the AIEC PA7E fractions. The SDS-PAGE was ran on an Invitrogen Nupage 4-12% Bis-Tris, 12 well gel, part number NP0322. The running buffer was Invitrogen Nupage MES SDS running buffer, part number NP0002. The gel was run with a voltage drop of 200V for 35 minutes. Lane 1 contained 5ul of the Invitrogen SeeBlue Plus2 ladder, part number LC5925. Lane 2 contained the resuspended PEG precipitate PA7E that was loaded onto the AIEC column. Lane 3 contained WT CPMV. Lanes 4-8 contained the target purified PA7E particles corresponding to the AIEC peak 1 that were collected and processed further. Lanes 9-10 contained the cleaved PA7E particles contaminate corresponding to the AIEC peak 2.

### Example 6 - Analysis of Inactivated Chimeric CPMV Virus Like Particles - Viral Genomic RNA Extraction.

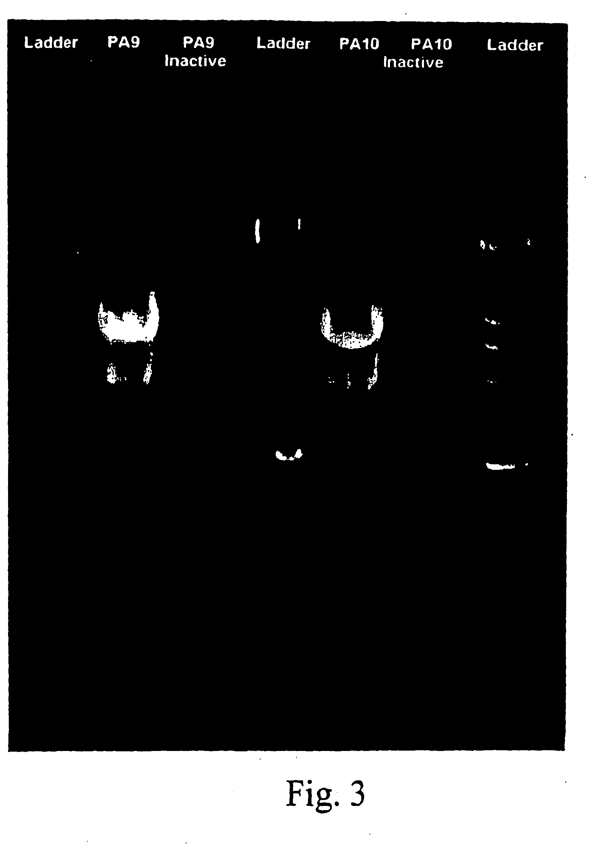

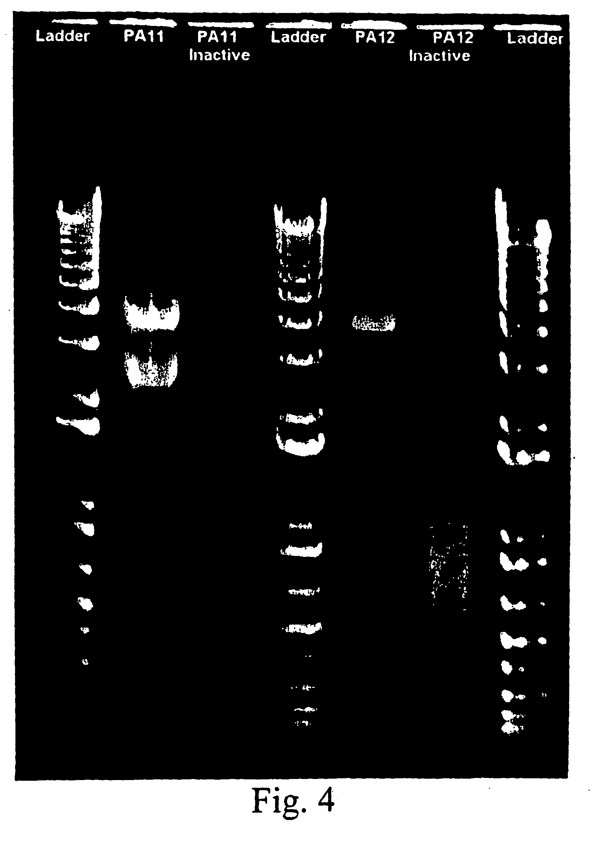

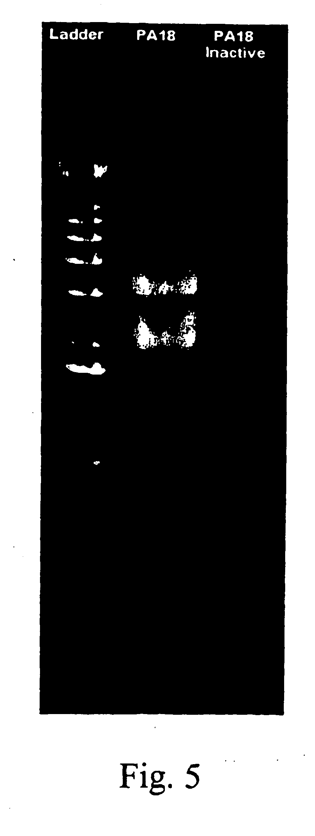

The Ambion RNAqueous, part number 1912, kit was used to extract the viral genomic RNA from the PEG purified inactivated CPMV samples. CPMV virus particles that had not been inactivated were used as a control (active samples). **Figures 3-5** show the results of RNA inactivation for PA9, PA10, PA11, PA12, and PA 18. All samples listed in the Example 3 were processed using the method described in the Example 6 with similar results. Precast 1.2% E-Gels from Invitrogen, part number G501801, were used to visualize the extracted RNA for Figures 1-3. All ladders in **Figures 3-5** were lul loads of 1 KB PLUS Ladder, part number 10787-026. In **Figures 3**, lane 1 is 1ul of ladder. Lane 2 is active PA9. Lane 3 is inactivated PA9. Lane 4 is ladder. Lane 5 is active PA10, lane 6 in inactive PA10. Lane 7 is ladder. In **Figures 4**, lane 1 is 1ul of ladder. Lane 2 is active PA11. Lane 3 is inactivated PA11. Lane 4 is ladder. Lane 5 is active PA12, lane 6 in inactive PA12. Lane 7 is ladder. In **Figures 5**, lane 1 is 1ul of ladder. Lane 2 is active PA18. Lane 3 is inactivated PA18. The viral genomic RNA was degraded in the inactivated samples as indicated by detection of "smear" but full-length CPMV genomic RNA1 and RNA2 was detected in samples that did not undergo inactivation.

### Example 7 - Analysis of Inactivated Chimeric CPMV Virus Like Particles - Stability by SDS-PAGE.

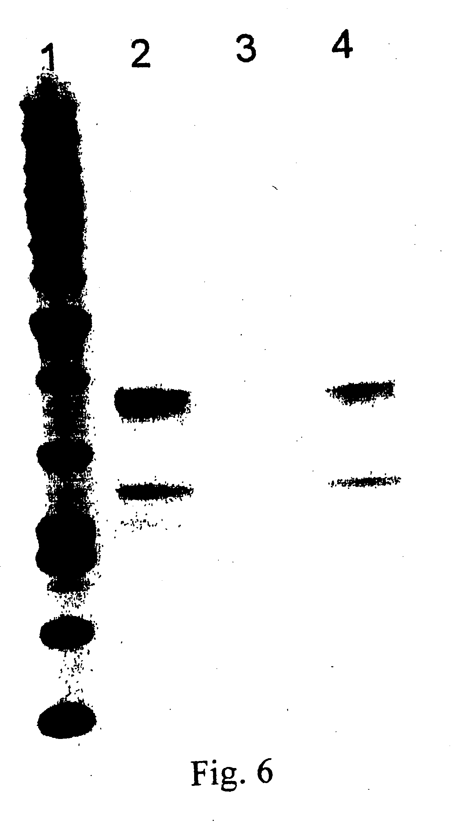

The stability of the small and large coat proteins were assayed with SDS-PAGE. **Figure 6** shows the SDS-PAGE gel of a 5 day temperature stability assay for PA1S as an example. The SDS-PAGE was ran on an Invitrogen Nupage 4-12% Bis-Tris, 12 well gel, part number NP0322. The gel was run with a voltage drop of 150V for 60 minutes. The running buffer was Invitrogen Nupage MES SDS running buffer, part number NP0002. Lane 1 contained 7ul of Invitrogen Benchmark Unstained Protein Ladder, part number 10747-012. Lane 2 contained inactivated PAIS virus particles incubated at room temperature for 5 days. Lane 3 contained inactivated PA1S virus particles incubated at 4° C for 5 days. Lane 4 contained inactivated PA1S virus particles incubated at -20C for 5 days. No protein degradation was detected.

### Example 8 - Analysis of Inactivated Chimeric CPMV Virus Like Particles - Stability by SEC.

The integrity of the assembled virus like particles was assayed using size exclusion chromatography (SEC). All samples listed in the Example 3 were analyzed using SEC with similar results. The SEC column used was a 30cm x 7.8mm Tosoh TskGel G5000 analytical SEC column from Supelco with 10 micron bead size, part number 08023. The mobile phase for the SEC was 0.1M NaPO4 pH 7.00. A single peak was detected corresponding to assembled virus particles. The assembled CPMV particles eluted from the column with a retention time of 14.0 minutes.

### Example 9 - Analysis of Inactivated Chimeric CPMV Virus Like Particles - Infectivity in Plants.

Inactivated chimeric CPMV particles listed in Example 3 were tested for their ability to infect plants. Cowpea California #5 seeds from Ferry Morse, part number 1450, were germinated over night at room temperature in wet paper towels. Germinated seeds were transferred into soil. Seven days post germination, ten seedlings were inoculated with inactivated and active WT or chimeric CPMV particles. After inoculation, the plants were grown at 25° C with a photo period of 16 hours light and 8 hours dark for two to three weeks and observed for symptom formation. Plants inoculated with inactivated WT or chimeric CPMV particles showed no symptoms but plants inoculated with active WT or chimeric CPMV particles showed typical symptoms of CPMV infection. Leaves inoculated with inactivated WT or chimeric CPMV particles were harvested and processed for virus particle isolation. 40g of leaf tissue was frozen at -80C. The frozen leaf tissue was crushed by hand and poured into a Waring high speed blender, part number 8011S. 120ml of cold 30mM Tris base, pH 7.50, 0.2mM PMSF was poured onto the crushed leaves. The leaves were ground 2 times for 3 seconds at high speed. The solution was decanted into a 500ml centrifuge bottle. The blender was washed with 30ml of cold buffer and the wash was poured into a 500ml centrifuge bottle. The solution was centrifuged at 15,000G for 30 minutes to remove the plant cellular debris. The supernatant was decanted into a graduated cylinder. To precipitate the CPMV virus, cold PEG 6000 solution (20% PEG 6000, 1M NaCl) was added to the supernatant to bring the final PEG concentration to 4% PEG 6000 with 0.2M NaCl, and the solution was gently mixed. The solution was allowed to precipitate for 1 hour on ice. The virus precipitate solution was then centrifuged at 15.000G for 30 minutes to collect virus particles in the pellet. The supernatant was poured off and the pellet was immediately resuspended in PBS buffer, pH 7.4. The samples were assayed for the presence of virus particle with SDS-PAGE. The SDS-PAGE was ran on an Invitrogen Nupage 4-12% Bis-Tris, 12 well gel, part number NP0322. The gel was run with a voltage drop of 150V for 60 minutes. The running buffer was Invitrogen Nupage MES SDS running buffer, part number NP0002. No virus particles were detected.

### Example 10 - Immunization of Mice with Inactivated CPMV Particles Containing PA Epitope.

Female Balb/c mice 7 weeks old were injected three times intraperitoneally with 100 µg purified inactivated CPMV-PA in the presence of adjuvant. Control mice received inactivated CPMV particles with unrelated peptide or only adjuvant in PBS, pH 7.0. 100 µl of Ribi adjuvant (R-700; Ribi Immunochem Research, Hamilton, Montana) mixed with 100 µl of the sample was used. Total volume for administration was 200 µl. The injections were given at 3-week intervals.

For intranasal immunization, inactivated CPMV-PA, without adjuvants, was administered to anesthetized mice. A total volume of 100 µl was administered in two nostrils (50 µl per each nostril). Control mice received inactivated CPMV with unrelated peptide or only PBS, pH 7.0.

Blood samples were obtained 1 day before the first administration and 2 weeks after each of the two subsequent administrations.

The summary of the mice immunization studies is provided below:

| **Table 4.** | | | | |
|---|---|---|---|---|
| **Adjuvant** | **Treatment** | **Route** | **Dose** | **# of mice** |
| Yes | CPMV-PA | IP | 3x 100 ug/200 ul | 5 |
| Yes | CPMV-control | IP | 3x 100 ug/200 ul | 5 |
| Yes | PBS, pH 7.0 | IP | 3xN/A/200 ul | 3 |
| No | CPMV-PA | IN | 3x 100 ug/100 ul | 5 |
| No | CPMV-control | IN | 3x 100 ug/100 ul | 5 |
| No | PBS, pH 7.0 | IN | 3xN/A/100 ul | 3 |

### Example 11 - Immunization of Non-Human Primates with Inactivated CPMV Particles Containing PA Epitopes.

The inactivated CPMV particles containing PA epitopes were tested for their ability to generate antibody responses to the co-expressed anthrax peptides when administered to rhesus macaques. Four monkeys were be immunized intra-muscularly with the inactivated CPMV-PA peptide constructs and one monkey with the inactivated wild type CPMV control. Each immunizing dose consisted of 2 mg of the virus-peptide mixture of all 16 PA-CPMV constructs. The animals were vaccinated at days 0, 7, 14, and 28.

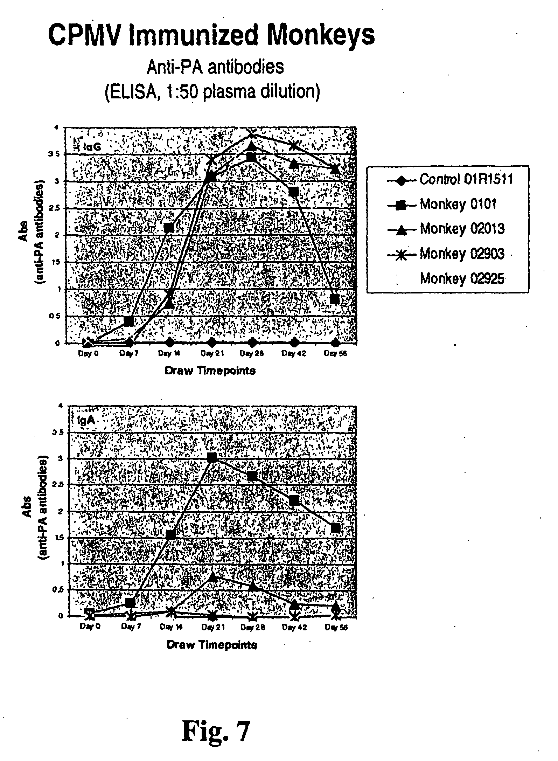

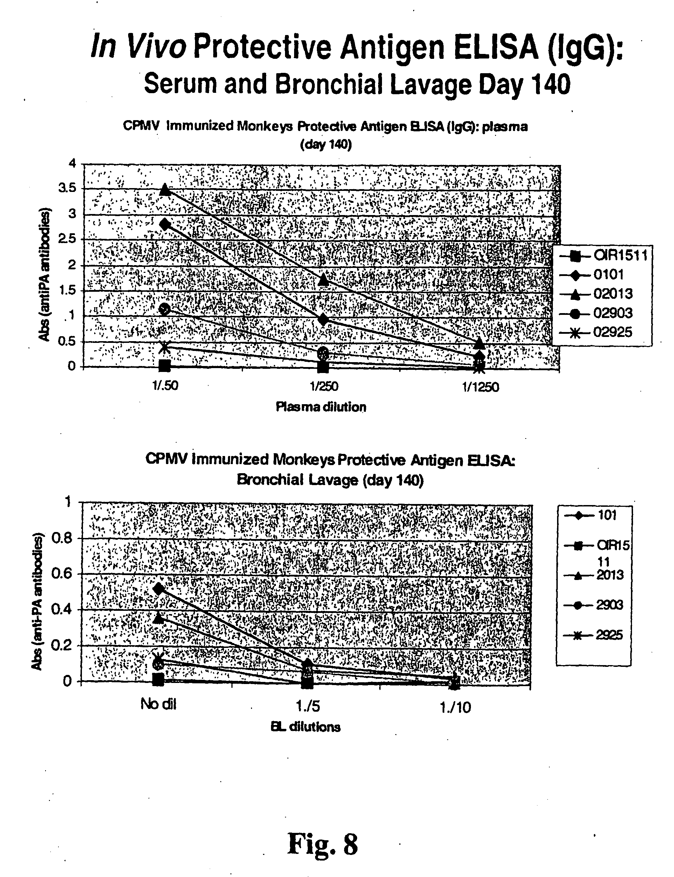

IgG and IgA antibodies were monitored using ELISA assays with PA protein as a target. Three to 5 ml of blood were drawn in heparin on each of the immunization days. Cells and plasma were separated and cryopreserved. Ketamine anesthesized monkeys were bronchoscoped on days 0, 14, and 28 and bronchial lavage specimens obtained and cryopreserved. The bronchial washings and plasma were thawed and IgG and IgA antibody titers measured in ELISA assays. High titres of both the IgG and IgA antibodies were detected in plasma and bronchial lavage. The results are shown in **Figure 7** **and** **8****.**

### Example 12 - Immunization of Mice with Inactivated CPMV Particles Containing Influenza Virus Epitope M2e

Female Balb/c mice 7 weeks old were injected three times intraperitoneally with 100 µg purified inactivated CPMV expressing an influenza peptide M2e in the presence of adjuvant. The sequence is SLLTEVETPIRNEGCRCNDSSD (SEQ ID NO: 24). Control mice received inactivated CPMV particles with unrelated peptide or only adjuvant in PBS, pH 7.0. 100 µl of Ribi adjuvant (R-700; Ribi Immunochem Research, Hamilton, Montana) mixed with 100 µl of the sample was used. Total volume for administration was 200 µl. The injections were given at 3-week intervals.

For intranasal immunization, inactivated CPMV containing an influenza peptide M2e, without adjuvants, were administered to anesthetized mice. A total volume of 100 µl was be administered in two nostrils (50 µl per each nostril). Control mice received inactivated CPMV with unrelated peptide or only PBS, pH 7.0.

Blood samples were obtained 1 day before the first administration and 2 weeks after each of the two subsequent administrations.

The summary of the mice immunization studies is provided below:

| **Table 6.** | | | | |
|---|---|---|---|---|
| **Adjuvant** | **Treatment** | **Route** | **Dose** | **# of mice** |
| Yes | CPMV-M2e | IP | 3x 100 ug/200 ul | 5 |
| Yes | CPMV-control | IP | 3x100 ug/200 ul | 5 |
| Yes | PBS, pH 7.0 | IP | 3xN/A/200 ul | 3 |
| No | CPMV-M2e | IN | 3x 100 ug/100 ul | 5 |
| No | CPMV-control | IN | 3x 100 ug/100 ul | 5 |
| No | PBS, pH 7.0 | IN | 3xN/A/100 ul | 3 |

The foregoing is illustrative of the present invention, and is not to be construed as limiting thereof The invention is defined by the following claims, with equivalents of the claims to be included therein.

## Claims

1. A method of inactivating a plant virus comprising:
administering ammonium sulfate to plant material expressing a virus-like particle wherein the plant material is selected from the group consisting of plants, plant tissue, plant cells and protoplasts at a pH above 8.0;
incubating the plant material for at least ten hours to produce an inactivated virus-like particle (VLP); and
harvesting the inactivated VLP from the plant material.

2. The method according to Claim 1, further comprising incorporating at least one foreign peptide into the virus.

3. The method according to Claim 2, wherein the foreign peptide incorporated into the virus is selected from the group consisting of a subunit of influenza virus, eastern equine encephalitis virus. Canine parvovirus, and *Bacillus anthracis.*

4. The method according to Claim 1, wherein said inactivated VLP presents a heterologous bioactive peptide.

5. The method according to any of Claims 2 to Claim 4, wherein the peptide is selected from the group consisting of a peptide hormone, an enzyme, a growth factor, an antibody, an immunoregulator, and a cytokine.

6. The method according to any of Claims 2 to Claim 4, wherein said peptide is an antigen or an epitope.

7. The method according to Claim 6, wherein the virus comprises coat proteins and the peptides are antigens fused to the coat proteins.

8. The method according to any of Claims 1 to 7, wherein said virus is a non-enveloped RNA virus.

9. The method according to Claim 8, wherein said method further comprises converting a viral RNA sequence into a full-length cDNA transcript, cloning said cDNA into a vector, and modifying said cDNA by inserting a foreign DNA segment in a region able to tolerate such insertion without disrupting RNA replication, particle formation, or infectivity.

10. The method according to any of Claims 1 to 9, wherein the ammonium sulfate is administered at a concentration of 0.5M to 1.0M.

11. The method according to any of Claims 1 to 10, wherein the pH is pH 9.0.

12. The method according to any of Claims 1 to 11, wherein the plant material is incubated between 10°C to 40°C.

13. The method according to any of Claims 2 to 12, wherein said method comprises binding said plant vitus to a hydrophobic interaction chromatography column in 0.7 M (NH₄)₂SO₄ at pH 7, washing bound virus with 0.7 M (NH₄)₂SO₄ at pH 9, and eluting said virus with 0.7 M (NH₄)₂SO₄ at pH 9.

14. The method according to any of Claims 1 to 13, wherein the virus has a capsid that is icosahedral.

15. The method according to any of Claims 1 to 14, wherein the virus is of a family selected from the group consisting of Bromoviridae, Comoviridae, and Tombusviridae.

16. The method according to Claim 15, wherein the virus is of a genus selected from the group consisting of *Bromovirus, Comovirus, Tombusvirus, Alfamovirus,* and *Sobeniovirus.*

17. The method according to Claim 16, wherein the virus is selected from the group consisting of cowpea mosaic virus, cowpea chlorotic mottle virus, tomato bushy stunt virus, alfalfa mosaic virus, brome mosaic virus, and southern bean mosaic virus.

18. A method of producing a non-infectious VLP comprising:
administering ammonium sulfate to plant material selected from the group consisting of plants, plant tissue, plant cells and protoplasts and lacks at least a portion of RNA present in a plant virus at a pH above 8.0;
incubating the plant material for at least ten hours; and
harvesting the inactivated VLP from the plant material, wherein said VLP is not capable of replicating.

## Patentansprüche

1. Verfahren zur Inaktivierung eines Pflanzenvirus, umfassend:
Verabreichen von Ammoniumsulfat an Pflanzenmaterial, das ein virusähnliches Partikel exprimiert, wobei das Pflanzenmaterial ausgewählt ist aus der Gruppe, bestehend aus Pflanzen, Pflanzengewebe, Pflanzenzellen und Protoplasten bei einem pH-Wert über 8,0;
Inkubieren des Pflanzenmaterials für mindestens zehn Stunden, um ein inaktiviertes virusähnliches Partikel (VLP) hervorzubringen; und
Ernten des inaktivierten VLP aus dem Pflanzenmaterial.

2. Verfahren nach Anspruch 1, des Weiteren umfassend:
Integrieren von mindestens einem fremden Peptid in das Virus.

3. Verfahren nach Anspruch 2, wobei das in das Virus integrierte fremde Peptid ausgewählt ist aus der Gruppe, bestehend aus einer Untereinheit des Influenzavirus, des östlichen Pferdeencephalitisvirus, des Hundeparovirus und des *Bacillus anthracis.*

4. Verfahren nach Anspruch 1, wobei das inaktivierte VLP ein heterologes bioaktives Peptid präsentiert.

5. Verfahren nach einem beliebigen der Ansprüche 2 bis 4, wobei das Peptid ausgewählt ist aus der Gruppe, bestehend aus einem Peptidhormon, einem Enzym, einem Wachstumsfaktor, einem Antikörper, einem Immunregulator und einem Zytokin.

6. Verfahren nach einem beliebigen der Ansprüche 2 bis 4, wobei das Peptid ein Antigen oder ein Epitop ist.

7. Verfahren nach Anspruch 6, wobei das Virus Hüllproteine umfasst und die Peptide Antigene sind, die an die Hüllproteine fusioniert sind.

8. Verfahren nach einem beliebigen der Ansprüche 1 bis 7, wobei das Virus ein unbehülltes RNA-Virus ist.

9. Verfahren nach Anspruch 8, wobei das Verfahren des Weiteren umfasst: Konvertieren einer viralen RNA-Sequenz in ein Volllängen-cDNA-Transkript, Klonieren der cDNA in einen Vektor und Modifizieren der cDNA durch Insertieren eines fremden DNA-Segments in einem Bereich, der in der Lage ist, eine solche Insertion zu tolerieren, ohne die RNA-Replikation, die Partikelbildung oder die Infektivität zu stören.

10. Verfahren nach einem beliebigen der Ansprüche 1 bis 9, wobei das Ammoniumsulfat in einer Konzentration von 0,5 M bis 1,0 M verabreicht wird.

11. Verfahren nach einem beliebigen der Ansprüche 1 bis 10, wobei der pH-Wert pH 9.0 beträgt.

12. Verfahren nach einem beliebigen der Ansprüche 1 bis 11, wobei das Pflanzenmaterial zwischen 10°C und 40°C inkubiert wird.

13. Verfahren nach einem beliebigen der Ansprüche 2 bis 12, wobei das Verfahren umfasst: Binden des Pflanzenvirus an eine hydrophobe Wechselwirkungschromatographiesäule in 0,7 M (NH₄)₂SO₄ bei pH 7, Waschen des gebundenen Virus mit 0,7 M (NH₄)₂SO₄ bei pH 9 und Eluieren des Virus mit 0,7 M (NH₄)₂SO₄ bei pH 9.

14. Verfahren nach einem beliebigen der Ansprüche 1 bis 13, wobei das Virus ein Capsid aufweist, das ikosaedrisch ist.

15. Verfahren nach einem beliebigen der Ansprüche 1 bis 14, wobei das Virus aus einer Familie stammt, die ausgewählt ist aus der Gruppe, bestehend aus Bromoviridae, Comoviridae und Tombusviridae.

16. Verfahren nach Anspruch 15, wobei das Virus aus einer Gattung stammt, die ausgewählt ist aus der Gruppe, bestehend aus *Bromovirus, Comovirus, Tombusvirus, Alfamovirus* und *Sobemovirus.*

17. Verfahren nach Anspruch 16, wobei das Virus ausgewählt ist aus der Gruppe, bestehend aus Kundebohnenmosaikvirus (Cowpea Mosaic Virus), chlorotischem Fleckenvirus der Kundebohne (Cowpea Chlorotic Mottle Virus), Tomatenzwergbuschvirus (Tomato Bushy Stunt Virus), Luzernemosaikvirus (Alfalfa Mosaic Virus), Trespenmosaikvirus (Brome Mosaik Virus) und südlichem Bohnenmosaikvirus (Southern Bean Mosaic Virus).

18. Verfahren zur Herstellung eines nichtinfektiösen VLP, umfassend:
Verabreichen von Ammoniumsulfat an Pflanzenmaterial, das ausgewählt ist aus der Gruppe, bestehend aus Pflanzen, Pflanzengewebe, Pflanzenzellen und Protoplasten, und dem zumindest ein Teil der RNA fehlt, die in einem Pflanzenvirus bei einem pH-Wert über 8,0 anwesend ist;
Inkubieren des Pflanzenmaterials für mindestens zehn Stunden; und
Ernten des inaktivierten VLP aus dem Pflanzenmaterial, wobei das VLP nicht zur Replikation fähig ist.

## Revendications

1. Procédé d'inactivation d'un virus de plante comprenant :
- l'administration de sulfate d'ammonium à un matériel végétal exprimant une particule de type virus, le matériel végétal étant choisi dans le groupe constitué par les plantes, le tissu végétal, les cellules végétales et les protoplastes à un pH supérieur à 8,0 ;
- l'incubation du matériel végétal pendant au moins dix heures pour produire une particule de type virus (VLP) inactivée ; et
- la récolte de la VLP inactivée à partir du matériel végétal.

2. Procédé selon la revendication 1, comprenant en outre l'incorporation d'au moins un peptide étranger dans le virus.

3. Procédé selon la revendication 2, dans lequel le peptide étranger incorporé dans le virus est choisi dans le groupe constitué par une sous-unité du virus de la grippe, le virus de l'encéphalite équine orientale, le parvovirus du chien et *Bacilles anthracis.*

4. Procédé selon la revendication 1, dans lequel ladite VLP inactivée présente un peptide bioactif hétérologue.

5. Procédé selon l'une quelconque des revendications 2 à 4, dans lequel le peptide est choisi dans le groupe constitué par une hormone peptidique, une enzyme, un facteur de croissance, un anticorps, un immunorégulateur et une cytokine.

6. Procédé selon l'une quelconque des revendications 2 à 4, dans lequel ledit peptide est un antigène ou un épitope.

7. Procédé selon la revendication 6, dans lequel le virus comprend des protéines d'enveloppe et les peptides sont des antigènes fusionnés aux protéines d'enveloppe.

8. Procédé selon l'une quelconque des revendications 1 à 7, dans lequel ledit virus est une virus à ARN non enveloppé.

9. Procédé selon la revendication 8, ledit procédé comprenant en outre la conversion d'une séquence d'ARN virale en un produit de transcription d'ADNc de longueur entière, le clonage dudit ADNc dans un vecteur, et la modification dudit ADNc par l'introduction d'un segment d'ADN étranger dans une région capable de tolérer une telle introduction sans interrompre la réplication de l'ARN, la formation de particules ou l'infectivité.

10. Procédé selon l'une quelconque des revendications 1 à 9, dans lequel le sulfate d'ammonium est administré à une concentration de 0,5 M à 1,0 M.

11. Procédé selon l'une quelconque des revendications 1 à 10, dans lequel le pH est un pH de 9,0.

12. Procédé selon l'une quelconque des revendications 1 à 11, dans lequel le matériel végétal est incubé entre 10°C et 40°C.

13. Procédé selon l'une quelconque des revendications 2 à 12, ledit procédé comprenant la liaison dudit virus de plante à une colonne de chromatographie à interaction hydrophobe dans du (NH₄)₂SO₄ 0,7 M à pH 7, le lavage du virus lié avec du (NH₄)₂SO₄ 0,7 M à pH 9, et l'élution dudit virus avec du (NH₄)₂SO₄ 0,7 M à pH 9.

14. Procédé selon l'une quelconque des revendications 1 à 13, dans lequel le virus a une capside qui est icosaédrique.

15. Procédé selon l'une quelconque des revendications 1 à 14, dans lequel le virus est d'une famille choisie dans le groupe constitué par les Bromoviridae, les Comoviridae et les Tombusviridae.

16. Procédé selon la revendication 15, dans lequel le virus est d'un genre choisi dans le groupe constitué par *Bromovirus, Comovirus, Tombusvirus, Alfamovirus* et *Sobemovirus.*

17. Procédé selon la revendication 16, dans lequel le virus est choisi dans le groupe constitué par le virus de la mosaïque du dolique, le virus des taches chlorotiques du dolique, le virus du rabougrissement buissonneux de la tomate, le virus de la mosaïque de la luzerne, le virus de la mosaïque du brome et le virus de la mosaïque du haricot du sud.

18. Procédé de production d'une VLP non infectieuse, comprenant :
- l'administration de sulfate d'ammonium à un matériel végétal choisi dans le groupe constitué par les plantes, le tissu végétal, les cellules végétales et les protoplastes et manquant au moins d'une partie de l'ARN présent dans un virus de plante à un pH supérieur à 8,0 ;
- l'incubation du matériel végétal pendant au moins dix heures ; et
- la récolte de la VLP inactivée à partir du matériel végétal, ladite VLP n'étant pas capable de se répliquer.
